(19) Europäisches Patentamt
European Patent Office
Office européen des brevets

(11) Veröffentlichungsnummer: **0 143 379**
**B1**

# (12) EUROPÄISCHE PATENTSCHRIFT

(45) Veröffentlichungstag der Patentschrift: **30.03.88**

(51) Int. Cl.⁴: **C 07 D 249/08**

(21) Anmeldenummer: **84113571.8**

(22) Anmeldetag: **10.11.84**

## (54) Verfahren zur Herstellung von beta-Hydroxyethyl-(1,2,4-triazol)-Derivaten.

(30) Priorität: **25.11.83 DE 3342692**

(43) Veröffentlichungstag der Anmeldung: **05.06.85 Patentblatt 85/23**

(45) Bekanntmachung des Hinweises auf die Patenterteilung: **30.03.88 Patentblatt 88/13**

(84) Benannte Vertragsstaaten: **BE CH DE FR GB IT LI NL**

(56) Entgegenhaltungen:
**DE - A - 3 018 865**
**DE - A - 3 102 588**
**DE - A - 3 202 604**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Reubke, Karl-Julius, Dr., Rybniker Strasse 10, D-5000 Köln 80 (DE)**

EP 0 143 379 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein neues Verfahren zur Herstellung von bekannten β-Hydroxyethyl-(1,2,4-triazol)-Derivaten, welche pflanzenwuchsregulierende und fungizide Eigenschaften besitzen.

Es ist bereits bekannt geworden, dass man β-Hydroxyethyl-(1,2,4-triazol)-Derivate herstellen kann, indem man Oxirane mit 1,2,4-Triazol in Gegenwart einer Base sowie in Gegenwart von inerten Lösungsmitteln, wie z.B. Methanol, Ethanol, n-Propanol, Acetonitril oder Dimethylformamid, umsetzt (vgl. EP-A-0 040 345, EP-A-0 044 605, EP-A-0 046 337 und EP-A-0 052 424). Nachteilig an diesem Verfahren ist jedoch, dass sich ausser den 1,2,4-Triazol-Derivaten je nach dem verwendeten Solvens mehr oder weniger grosse Mengen an 1,3,4-Triazol-Derivaten bilden. Arbeitet man beispielsweise in Gegenwart von Alkoholen, so entstehen bis zu 30% an den unerwünschten Stoffen. Die Abtrennung dieser störenden Nebenprodukte ist aufwendig, und die Ausbeuten an den gewünschten 1,2,4-Triazol-Derivaten liegen daher nur relativ niedrig. Ungünstig an diesem bekannten Verfahren ist auch, dass verhältnismässig lange Reaktionszeiten erforderlich sind.

Es wurde nun gefunden, dass man die bekannten β-Hydroxyethyl-(1,2,4-triazol)-Derivate der Formel

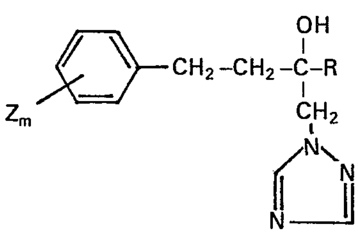

(I)

in welcher

R für Alkyl, Halogenalkyl, gegebenenfalls substituiertes Cycloalkyl oder gegebenenfalls substituiertes Phenyl steht,

Z für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl oder gegebenenfalls substituiertes Phenylalkoxy steht und

m für die Zahlen 0, 1, 2 oder 3 steht, durch Umsetzung von Oxiranen der Formel

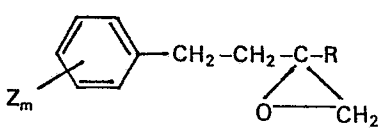

(II)

in welcher

R, Z und m die oben angegebene Bedeutung haben,

mit 1,2,4-Triazol der Formel

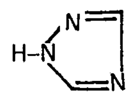

(III)

erhält, wenn man die Umsetzung der Oxirane der Formel (II) mit 1,2,4-Triazol in Gegenwart von cyclischen Amiden der Formel

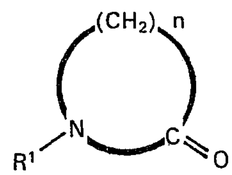

(IV)

in welcher

$R^1$ für Alkyl mit 1 bis 4 Kohlenstoffatomen steht und

n für die Zahlen 3, 4 oder 5 steht,

als Verdünnungsmittel und in Gegenwart einer Base sowie gegebenenfalls in Gegenwart von Azo-bis-isobutyronitril, Benzo-yl-peroxid, Luft oder Sauerstoff als Co-Katalysator und gegebenenfalls in Gegenwart von Wasser bei Temperaturen zwischen 0 °C und 200 °C durchführt.

Es ist als äusserst überraschend zu bezeichnen, dass sich die β-Hydroxyethyl-(1,2,4-triazol)-Derivate der Formel (I) nach dem erfindungsgemässen Verfahren in höheren Ausbeuten herstellen lassen als nach der bisher bekannten Methode. Insbesondere konnte nicht erwartet werden, dass die Bildung unerwünschter 1,3,4-Triazol-Derivate speziell beim Arbeiten in Gegenwart von cyclischen Amiden der Formel (IV) unterbleibt.

Das erfindungsgemässe Verfahren besitzt eine Reihe von Vorteilen. So ermöglicht es die Herstellung von β-Hydroxyethyl-(1,2,4-triazol)-Derivaten der Formel (I) in sehr guten Ausbeuten. Ausserdem sind die Reaktionszeiten erheblich kürzer als bei dem bisher bekannten Verfahren zur Synthese der Verbindungen der Formel (I). Darüber hinaus bereitet die Isolierung der erfindungsgemäss herstellbaren Produkte aus dem bei der Umsetzung anfallenden Reaktionsgemisch keine Schwierigkeiten. Vor allem sind keine aufwendigen Umkristallisationen erforderlich.

Verwendet man 2-(4-Chlor-phenyl-ethyl)-2-tert.-butyloxiran und 1,2,4-Triazol als Ausgangsstoffe und N-Methylpyrrolidon als Lösungsmittel, so kann der Ablauf des erfindungsgemässen Verfahrens durch das folgende Formelschema veranschaulicht werden:

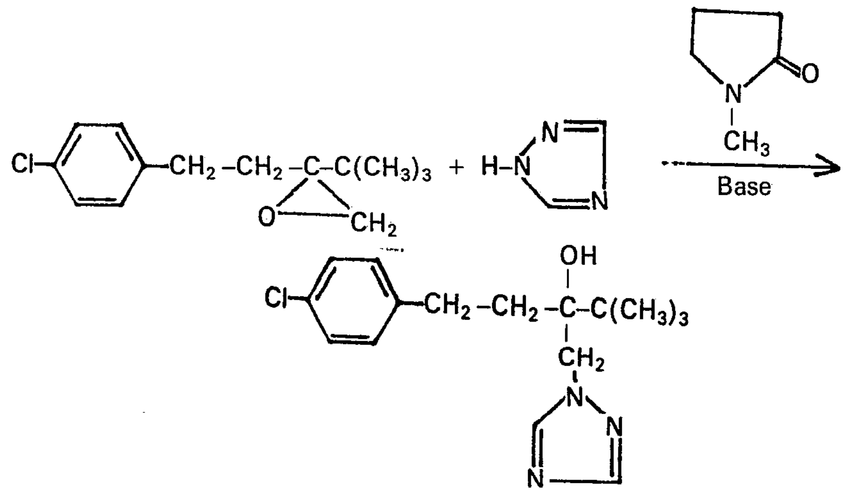


Die bei dem erfindungsgemässen Verfahren als Ausgangsstoffe zu verwendenden Oxirane sind durch die Formel (II) allgemein definiert. In dieser Formel steht R vorzugsweise für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 5 Halogenatomen, gegebenenfalls durch Alkyl mit 1 bis 2 Kohlenstoffatomen, substituiertes Cycloalkyl mit 3 bis 7 Kohlenstoffatomen sowie für gegebenenfalls einfach oder mehrfach, gleich oder verschieden substituiertes Phenyl, wobei als Substituenten vorzugsweise in Frage kommen: Halogen, Alkyl mit 1 bis 4 Kohlenstoffatomen sowie Halogenalkyl mit 1 bis 2 Kohlenstoff- und 1 bis 5 gleichen oder verschiedenen Halogenatomen, wie insbesondere Fluor- und Chloratomen. Z steht vorzugsweise für Halogen, geradkettiges oder verzweigtes Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie insbesondere Fluor- und Chloratomen sowie für jeweils gegebenenfalls substituiertes Phenyl, Phenoxy und Phenylakyl sowie Phenylalkoxy mit 1 bis 2 Kohlenstoffatomen im Alkylteil bzw. im Alkoxyteil, wobei als Substituenten vorzugsweise genannt seien: Halogen und Alkyl mit 1 bis 4 Kohlenstoffatomen. Der Index m steht für die Zahlen 0, 1, 2 oder 3, wobei die für Z in Frage kommenden Reste gleichartig und verschieden sein können.

Besonders bevorzugt sind diejenigen Verbindungen der Formel (II), in denen R für Methyl, Isopropyl, tert.-Butyl, durch Fluor und/oder Chlor substituiertes Methyl, Isopropyl oder tert.-Butyl steht, ferner für gegebenenfalls durch Methyl substistuiertes Cyclopropyl, gegebenenfalls durch Methyl substituiertes Cyclopentyl oder gegebenenfalls durch Methyl substituiertes Cyclohexyl steht, sowie für gegebenenfalls einfach oder zweifach, gleichartig oder verschieden durch Fluor, Chlor, Methyl und/oder Trifluormethyl substituiertes Phenyl steht, Z für Fluor, Chlor, Brom, Methyl, tert.-Butyl Cyclohexyl, Methoxy, Methylthio, Trifluormethyl, Trifluormethoxy, Trifluormethylthio sowie für jeweils gegebenenfalls einfach oder zweifach, gleich oder verschieden durch Fluor, Chlor und/oder Methyl substituiertes Phenyl, Phenoxy, Benzyl oder Benzyloxy steht, und m für die Zahlen 0, 1, 2 oder 3 steht, wobei die für Z in Betracht kommenden Reste gleichartig oder verschieden sein können.

Als Beispiele für Oxirane der Formel (II) seien die in der folgenden Tabelle formelmässig aufgeführten Stoffe genannt.

Tabelle 1

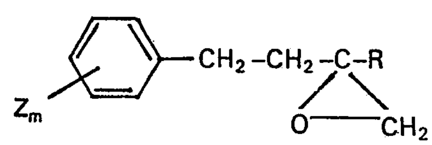 (II)

| $Z_m$ | R |
|---|---|
| 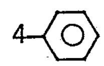 | $-C(CH_3)_3$ |
| 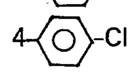 | do. |
| 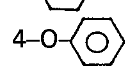 | do. |
| 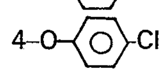 | do. |
| 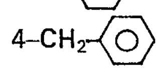 | do. |
| 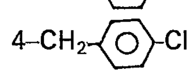 | do. |
| 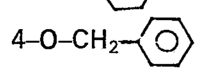 | do. |
| 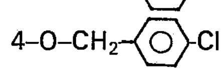 | do. |
| 3,4–$Cl_2$ | do. |
| 4–$CF_3$ | do. |
| 4–$OCF_3$ | do. |
| 4–$SCF_3$ | do. |
| 4–$SCH_3$ | do. |
| 4–$C(CH_3)_3$ | do. |

Tabelle 1 (Fortsetzung)

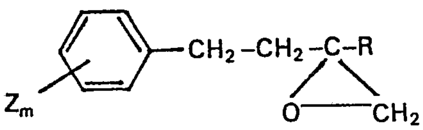 (II)

| $Z_m$ | R |
|---|---|
| 4–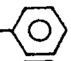 | 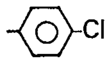 |
| 4–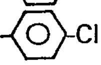 | do. |
| 4–O–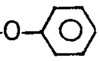 | do. |
| 4–O–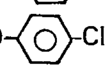 | do. |
| 4–$CH_2$–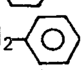 | do. |
| 4–$CH_2$–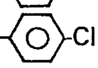 | do. |
| 4–O–$CH_2$–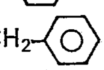 | do. |
| 4–O–$CH_2$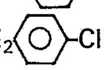 | do. |
| 3,4–$Cl_2$ | do. |
| 4–$CF_3$ | do. |
| 4–$OCF_3$ | do. |
| 4–$SCF_3$ | do. |
| 4–$SCH_3$ | do. |
| 4–$C(CH_3)_3$ | do. |

Tabelle 1 (Fortsetzung)

| $Z_m$ | R |
|---|---|
| 4–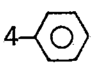 | $-CH(CH_3)_2$ |
| 4–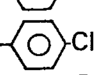 | do. |
| 4–O–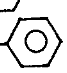 | do. |
| 4–O–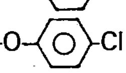 | do. |
| 4–$CH_2$–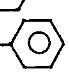 | do. |
| 4–$CH_2$–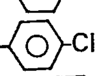 | do. |
| 4–O–$CH_2$–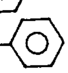 | do. |
| 4–O–$CH_2$–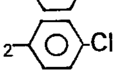 | do. |
| 3,4–$Cl_2$ | do. |
| 4–$CF_3$ | do. |
| 4–$OCF_3$ | do. |
| 4–$SCF_3$ | do. |
| 4–$SCH_3$ | do. |
| 4–$C(CH_3)_3$ | do. |

| | |
|---|---|
| 4–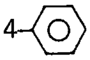 | 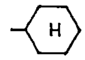 |
| 4–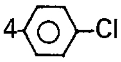 | do. |
| 4–O–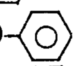 | do. |
| 4–O–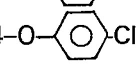 | do. |
| 4–$CH_2$–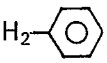 | do. |
| 4–$CH_2$–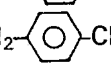 | do. |
| 4–O–$CH_2$–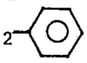 | do. |
| 4–O–$CH_2$–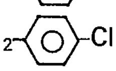 | do. |
| 3,4–$Cl_2$ | do. |
| 4–$CF_3$ | do. |
| 4–$OCF_3$ | do. |
| 4–$SCF_3$ | do. |
| 4–$SCH_3$ | do. |
| 4–$C(CH_3)_3$ | do. |

Tabelle 1 (Fortsetzung)

| $Z_m$ | R |
|---|---|
| 4–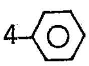 | 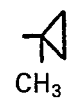 |
| 4–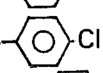 | do. |
| 4–O–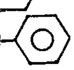 | do. |
| 4–O–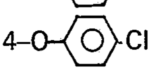 | do. |
| 4–$CH_2$–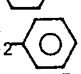 | do. |
| 4–$CH_2$–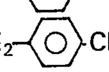 | do. |
| 4–O–$CH_2$–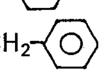 | do. |
| 4–O–$CH_2$–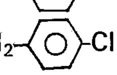 | do. |
| 3,4–$Cl_2$ | do. |
| 4–$CF_3$ | do. |
| 4–$OCF_3$ | do. |
| 4–$SCF_3$ | do. |
| 4–$SCH_3$ | do. |
| 4–$C(CH_3)_3$ | do. |
| 4–Cl | $-CH(CH_3)_2$ |
| 4–F | do. |
| 4–$CH_3$ | do. |

Tabelle 1 (Fortsetzung)

| $Z_m$ | R |
|---|---|
| 4–Cl | 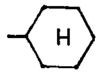 |
| 4–F | do. |
| 4–$CH_3$ | do. |
| 4–Cl | 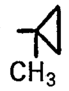 |
| 4–F | do. |
| 4–$CH_3$ | do. |
| 2,4–$Cl_2$ | –$C(CH_3)_3$ |
| 4–$CH_3$ | do. |
| 4–Cl, 2–$CH_3$ | do. |
| 2–$CH_3$ | do. |

Die Oxirane der Formel (II) sind bekannt oder lassen sich nach bekannten Methoden in einfacher Weise herstellen (vgl. EP-A-0 040 345, EP-A-0 046 337 und EP-A-0 052 424.

Die bei dem erfindungsgemässen Verfahren als Verdünnungsmittel fungierenden cyclischen Amide sind durch die Formel (IV) definiert.

In dieser Formel steht $R^1$ vorzugsweise für Methyl oder Ethyl und n steht für die Zahlen 3, 4 oder 5.

Als Beispiele für cyclische Amide der Formel (IV) seien genannt: N-Methyl-pyrrolidon, N-Ethyl-pyrrolidon, N-Methyl-hexahydro-pyridon und N-Methyl-ε-caprolactam.

Die cyclischen Amide der Formel (IV) sind bereits bekannt.

Als Basen kommen für die erfindungsgemässe Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Besonders bevorzugt sind Natrium- und Kaliumhydroxid und -carbonat.

Das erfindungsgemässe Verfahren wird vorzugsweise in Gegenwart eines der genannten Co-Katalysatoren durchgeführt.

In manchen Fällen ist es vorteilhaft, die erfindungsgemässe Umsetzung in Gegenwart einer geringen Menge an Wasser durchzuführen, um so eine bessere Löslichkeit der eingesetzten Base zu erzielen.

Die Reaktionstemperaturen können bei der Durchführung des erfindungsgemässen Verfahrens in einem grösseren Bereich variiert werden. Man arbeitet bei Temperaturen zwischen 0 und 200 °C, vorzugsweise zwischen 60 und 150 °C.

Das erfindungsgemässe Verfahren wird im allgemeinen unter Normaldruck durchgeführt. Es ist jedoch auch möglich, unter erhöhtem oder vermindertem Druck zu arbeiten.

Bei der Durchführung des erfindungsgemässen Verfahrens setzt man auf 1 Mol Oxiran der Formel (II) vorzugsweise keinen Überschuss, sondern 1 Mol an 1,2,4-Triazol sowie 0,1 bis 0,5 Mol an Base und eine ausreichende Menge an cyclischem Amid der Formel (IV) als Lösungsmittel ein. Verwendet man einen Co-Katalysator, so wird dieser in Mengen von 0,01 bis 1 Gew.-%, bezogen auf eingesetztes Oxiran der Formel (II), zugegeben. Verwendet man Wasser als Hilfslösungsmittel, so wird dieses nur in Spuren zugesetzt. Im allgemeinen verwendet man Wasser in Mengen von 0,1 bis 2 Gew.-%, bezogen auf die eingesetzte Lösungsmittelmenge. Die erfindungsgemässe Umsetzung ist im allgemeinen innerhalb von 2 bis 5 Stunden beendet. Die Aufarbeitung erfolgt nach üblichen Methoden. Im allgemeinen geht man so vor, dass man das Reaktionsgemisch durch Abdestillieren des Lösungsmittels einengt, den verbleibenden Rückstand mit Wasser wäscht, dann trocknet und mit einem inerten organischen Lösungsmittel, wie z.B. Petrolether oder Benzin, verrührt. Das sich dabei abscheidende Produkt wird abgetrennt und erneut getrocknet.

Die nach dem erfindungsgemässen Verfahren herstellbaren β-Hydroxyethyl-(1,2,4-triazol)-Derivate der Formel (I) sind bekannt (vgl. EP-A-0 040 345). Sie zeichnen sich durch sehr gute pflanzenwuchsregulierende und fungizide Eigenschaften aus.

Das erfindungsgemässe Verfahren wird durch die nachfolgenden Beispiele veranschaulicht.

Beispiel 1

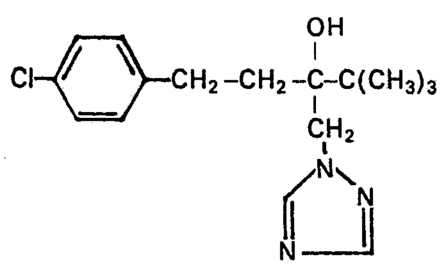

(I-1)

In eine Lösung von 71,55 g (0,3 Mol) 2-tert.-Butyl-2-(4-chlor-phenyl-ethyl)-oxiran in 10 ml N-Methylpyrrolidon wurden 4,15 g (0,103 Mol) Natriumhydroxid und eine Spatelspitze Azo-bis-isobutyronitril gegeben. Man heizte auf 120 °C auf und tropfte unter Rühren innerhalb von 3 Stunden eine Lösung von 20,7 g (0,3 Mol) 1,2,4-Triazol hinzu. Anschliessend rührte man noch eine Stunde bei 120 °C und liess dann abkühlen. Danach arbeitete man auf, indem man das N-Methyl-pyrrolidon bei einer Badtemperatur von 100 °C unter einem Druck von 0,2 mbar abdestillierte, den verbleibenden Rückstand mit Wasser wusch, dann trocknete und mit 150 ml Benzin verrührte. Das sich abscheidende Festprodukt wurde abfiltriert und erneut getrocknet. Man erhielt auf diese Weise 78,9 g (85,8% der Theorie) an 1-(4-Chlor-phenyl)-4,4-dimethyl-3-(1,2,4-triazol-1-yl-methyl)-pentan-3-ol.

Durch HPLC-Analyse wurde nachgewiesen, dass das Produkt nicht durch das entsprechende 1,3,4-Triazol-Derivat verunreinigt ist.

Beispiel 2

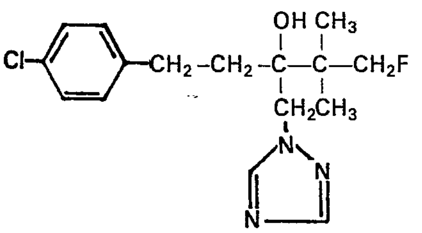

(I-2)

Nach der im Beispiel 1 angegebenen Methode wurde ausgehend von 2-(4-Chlor-phenyl--ethyl)-2-(mono-fluor-tert.-Butyl)-oxiran das 1-(4-Chlor-phenyl)-3- (1,2,4-triazol- 1-yl- methyl)-4,4-dimethyl-5-fluor-pentan-3-ol erhalten. Ausbeute: 84,7%

Beispiel 3

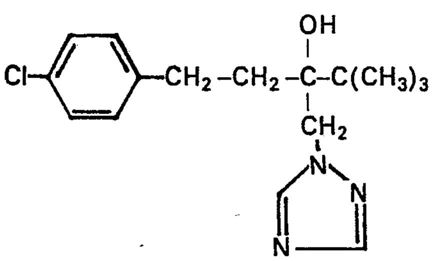

(I-1)

In eine Lösung von 71,55 g (0,3 Mol) 2-tert.-Butyl-2-(4-chlor-phenyl-ethyl)-oxiran in 125 ml N-Methylpyrrolidon wurden bei Raumtemperatur unter Rühren 20,7 g (0,3 Mol) 1,2,4-Triazol, 4,15 g (0,103 Mol) Natriumhydroxid und 1 ml Wasser gegeben. Man heizte unter Rühren 4 Stunden lang auf 120 °C und leitete währenddessen einen trockenen und $CO_2$-freien Luftstrom langsam durch das Reaktionsgemisch. Danach arbeitete man auf, indem man das Lösungsmittel unter vermindertem Druck abdestillierte, den verbleibenden Rückstand mit Wasser wusch, dann trocknete und mit 150 ml Benzin verrührte. Das sich abscheidende Festprodukt wurde abfiltriert und erneut getrocknet. Man erhielt auf diese Weise 81,9 g (88,8% der Theorie) an 1-(4-Chlorphenyl)- 4,4-dimethyl- 3-(1,2,4- triazol-1-yl-methyl)-pentan-3-ol.

Durch HPLC-Analyse wurde nachgewiesen, dass das Produkt nicht durch das entsprechende 1,3,4-Triazol-Derivat verunreinigt ist.

Vergleichsbeispiel

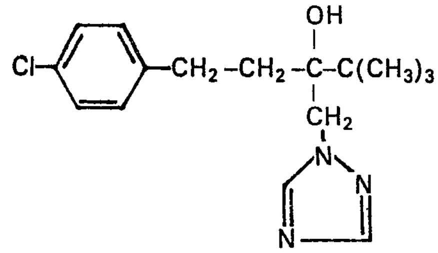

(I-1)

Eine Lösung von 27,1 g (0,1 Mol) eines Produktes, das zu 88% aus 2-(4-Chlor-phenyl-ethyl)-2-tert.-butyl-oxiran bestand, 8,3 g (0,12 Mol) 1,2,4-Triazol und 0,6 g (0,01 Mol) Kaliumhydroxid in 100 ml n-Propanol wurde 30 Stunden auf 95 °C erhitzt. Danach liess man abkühlen und engte das Reaktionsgemisch durch Abziehen des Lösungsmittels unter vermindertem Druck ein. Der verbliebene Rückstand wurde in Toluol aufgenommen, die dabei entstehende Suspension wurde filtriert, und das Filtrat wurde durch Abziehen des Lösungsmittels unter vermindertem Druck eingeengt. Der anfallende Rückstand wurde aus Ligroin umkristallisiert. Man erhielt auf diese Weise 30,6 g eines Produktes, das gemäss HPLC-Analyse zu 67,4% aus 1-(4-Chlorphenyl)-4,4-dimethyl-3-(1,2,4-triazol-1-yl-methyl)-pentan-3-ol bestand. Danach errechnet sich eine Ausbeute von 67% der Theorie.

**Patentansprüche**

1. Verfahren zur Herstellung von β-Hydroxyethyl-(1,2,4-triazol)-Derivaten der Formel

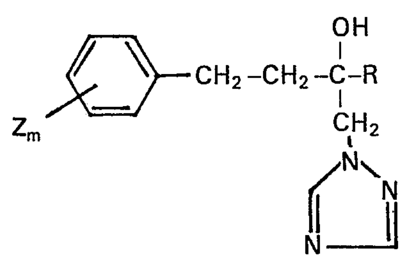

(I)

in welcher

R für Alkyl, Halogenalkyl, gegebenenfalls substituiertes Cycloalkyl oder gegebenenfalls substituiertes Phenyl steht,

Z für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl oder gegebenenfalls substituiertes Phenylalkoxy steht und

m für die Zahlen 0, 1, 2 oder 3 steht,

durch Umsetzung von Oxiranen der Formel

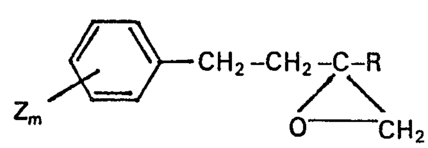

(II)

in welcher

R, Z und m die oben angegebene Bedeutung haben, mit 1,2,4-Triazol der Formel

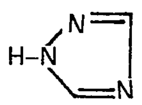

(III)

dadurch gekennzeichnet, dass man die Umsetzung der Oxirane der Formel (II) mit 1,2,4-Triazol in Gegenwart von cyclischen Amiden der Formel

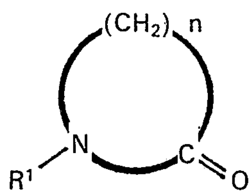

(IV)

in welcher

$R^1$ für Alkyl mit 1 bis 4 Kohlenstoffatomen steht und

n für die Zahlen 3, 4 oder 5 steht,

als Verdünnungsmittel und in Gegenwart einer Base sowie gegebenenfalls in Gegenwart von Azo-bis-isobutyronitril, Benzo-yl-peroxid, Luft oder Sauerstoff als Co-Katalysator und gegebenenfalls in Gegenwart von Wasser bei Temperaturen zwischen 0 °C und 200 °C durchführt.

2. Verfahren gemäss Anspruch 1, dadurch gekennzeichnet, dass man Oxirane der Formel (II) einsetzt, in denen

R für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 5 Halogenatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

wobei die für Z in Frage kommenden Reste gleichartig oder verschieden sein können.

3. Verfahren gemäss Anspruch 1, dadurch gekennzeichnet, dass man als Oxiran der Formel (II) das 2-tert.-Butyl-2-(4-chlor-phenyl-ethyl)-oxiran einsetzt.

4. Verfahren gemäss Anspruch 1, dadurch gekennzeichnet, dass man als cyclische Amide Verbindungen der Formel (IV) einsetzt, in denen

$R^1$ für Methyl oder Ethyl steht und

n für die Zahlen 3, 4 oder 5 steht.

5. Verfahren gemäss Anspruch 1, dadurch gekennzeichnet, dass man als cyclische Amide der Formel (IV) N-Methyl-pyrrolidon, N-Ethyl-pyrrolidon, N-Methyl-hexyhydropyridon oder N-Methyl-ε-caprolactam einsetzt.

6. Verfahren gemäss Anspruch 1, dadurch gekennzeichnet, dass man als Basen Alkalicarbonate, Alkalihydroxide, Alkalialkoholate, Alkalihydride, niedere tertiäre Alkylamine, Cycloalkylamine oder Aralkylamine einsetzt.

7. Verfahren gemäs Anspruch 1, dadurch gekennzeichnet, dass man Azo-bis-isobutyronitril, Benzoylperoxid, Luft oder Sauerstoff als Co-Katalysatoren einsetzt.

8. Verfahren gemäss Anspruch 1, dadurch gekennzeichnet, dass man Wasser als Hilfslösungsmittel einsetzt.

9. Verfahren gemäss Anspruch 1, dadurch gekennzeichnet, dass man die Umsetzung bei Temperaturen zwischen 60 °C und 150 °C durchführt.

## Revendications

1. Procédé de préparation de dérivés de β-hydroxyéthyl-(1,2,4-triazole) de formule:

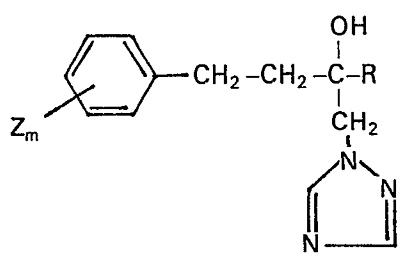

(I)

dans laquelle

R représente un groupe alkyle, un groupe halogénalkyle, un groupe cycloalkyle éventuellement substitué ou un groupe phényle éventuellement substitué,

Z représente un atome d'halogène, un groupe alkyle, un groupe cycloalkyle, un groupe alcoxy, un groupe alkylthio, un groupe halogénalkyle, un groupe halogénalcoxy, un groupe halogénalkylthio, un groupe phényle éventuellement substitué, un groupe phénoxy éventuellement substitué, un groupe phénylalkyle éventuellement substitué, ou un groupe phénylalcoxy éventuellement substitué, et

m représente les nombres 0, 1, 2 ou 3, par réaction d'oxyranes de formule:

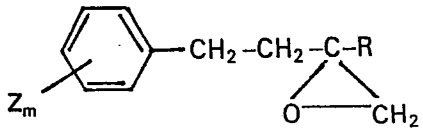

(II)

dans laquelle

R, Z et m ont les significations indiquées ci-dessus, avec du 1,2,4-triazole de formule:

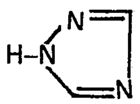

(III)

caractérisé en ce qu'on effectue la réaction des oxyranes de formule (II) avec du 1,2,4-triazole en présence d'amides cycliques de formule:

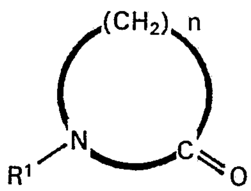

(IV)

dans laquelle

$R^1$ représente un groupe alkyle contenant 1 à 4 atomes de carbone, et

n représente les nombres 3, 4 ou 5, comme diluants et en présence d'une base, ainsi qu'éventuellement en présence d'azo-bis-isobutyro-nitrile, de peroxyde de benzoyle, d'air ou d'oxygène comme co-catalyseur et éventuellement en présence d'eau, à des températures comprises entre 0 °C et 200 °C.

2. Procédé selon la revendication 1, caractérisé en ce qu'on utilise des oxyranes de formule (II), dans laquelle

R représente un groupe alkyle à chaîne droite ou ramifiée contenant 1 à 4 atomes de carbone, un groupe halogénalkyle à chaîne droite ou ramifiée contenant 1 à 4 atomes de carbone et 1 à 5 atomes d'halogènes, un groupe cycloalkyle contenant 3 à 7 atomes de carbone et éventuellement substitué par un groupe alkyle contenant 1 ou 2 atomes de carbone, ou un groupe phényle éventuellement substitué par un atome d'halogène, par un groupe alkyle contenant 1 à 4 atomes de carbone et/ou par un groupe halogénalkyle contenant 1 ou 2 atomes de carbone et 1 à 5 atomes d'halogènes,

Z représente un atome d'halogène, un groupe alkyle contenant 1 à 4 atomes de carbone, un groupe cycloalkyle contenant 5 à 7 atomes de carbone, un groupe alcoxy contenant 1 à 4 atomes de carbone, un groupe alkylthio contenant 1 à 4 atomes de carbone, un groupe halogénalcoxy contenant 1 ou 2 atomes de carbone et 1 à 5 atomes d'halogènes, un groupe halogénalkylthio contenant 1 ou 2 atomes de carbone et 1 à 5 atomes d'halogènes, un groupe phényle éventuellement substitué par 1 atome d'halogène et/ou par un groupe alkyle contenant 1 à 4 atomes de carbone, un groupe phénoxy éventuellement substitué par un atome d'halogène et/ou par un groupe alkyle contenant 1 à 4 atomes de carbone, un groupe phénylalkyle contenant 1 ou 2 atomes de carbone dans la fraction alkyle et éventuellement substitué par un atome d'halogène et/ou par un groupe alkyle contenant 1 à 4 atomes de carbone, ou un groupe phénylalcoxy contenant 1 ou 2 atomes de carbone dans la fraction alcoxy et éventuellement substitué par un atome d'halogène et/ou par un groupe alkyle contenant 1 à 4 atomes de carbone, et

m représente les nombres 0, 1, 2 ou 3, les radicaux envisagés pour Z pouvant être de nature identique ou différente.

3. Procédé selon la revendication 1, caractérisé en ce que, comme oxyrane de formule (II), on utilise le 2-tert.-butyl-2-(4-chloro-phényl-éthyl)-oxyrane.

4. Procédé selon la revendication 1, caractérisé en ce que, comme amides cycliques, on utilise des composés de formule (IV) dans laquelle

$R^1$ représente le groupe méthyle ou le groupe éthyle, et

m représente les nombres 3, 4 ou 5.

5. Procédé selon la revendication 1, caractérisé en ce que, comme amides cycliques de formule (IV), on utilise la N-méthyl-pyrrolidone, la N-éthyl-pyrrolidone, la N-méthyl-hexahydropyridone ou le N-méthyl-ε-caprolactame.

6. Procédé selon la revendication 1, caractérisé en ce que, comme bases, on utilise des carbonates de métaux alcalins, des hydroxydes de métaux alcalins, des alcoolates de métaux alcalins, des hydrures de métaux alcalins, des alkylamines tertiaires inférieures, des cycloalkylamines ou des aralkylamines.

7. Procédé selon la revendication 1, caractérisé en ce qu'on utilise l'azo-bis-isobutyronitrile, le peroxyde de benzoyle, l'air ou l'oxygène, comme co-catalyseurs.

8. Procédé selon la revendication 1, caractérisé en ce qu'on utilise l'eau comme solvant auxiliaire.

9. Procédé selon la revendication 1, caractérisé en ce qu'on effectue la réaction à des températures comprises entre 60 °C et 150 °C.

**Claims**

1. Process for the preparation of β-hydroxyethyl-(1,2,4-triazole) derivatives of the formula

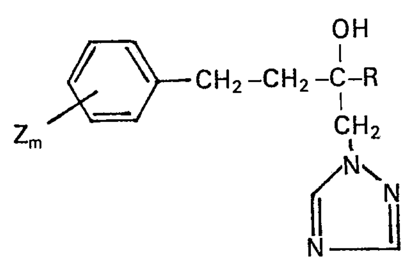

(I)

in which

R represents alkyl, halogenoalkyl, optionally substituted cycloalkyl or optionally substituted phenyl,

Z represents halogen, alkyl, cycloalkyl, alkoxy, alkylthio, halogenoalkyl, halogenoalkoxy, halogenoalkylthio, optionally substituted phenyl, optionally substituted phenoxy, optionally substituted phenylalkyl or optionally substituted phenylalkoxy and

m represents the number 0, 1, 2 or 3, by reacting oxiranes of the formula

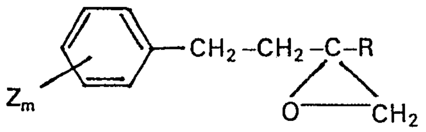

(II)

in which

R, Z and m have the abovementioned meaning, with 1,2,4-triazole of the formula

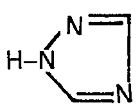

(III)

characterised in thath the reaction of the oxiranes of the formula (II) with 1,2,4-triazole is carried out in the presence of cyclic amides of the formula

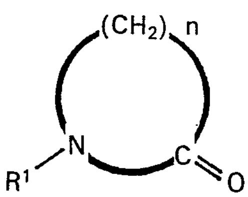

(IV)

in which

$R^1$ represents alkyl with 1 to 4 carbon atoms and

n represents the number 3, 4 or 5, as diluents, and in the presence of a base and, if appropriate, in the presence of azo-bis-isobutyronitrile, benzoyl peroxide, air or oxygen as co-catalyst and if appropriate in the presence of water, at temperatures between 0 °C and 200 °C.

2. Process according to claim 1, characterised in that oxiranes of the formula (II)

in which

R represents straight-chain or branched alkyl with 1 to 4 carbon atoms, straight-chain or branched halogenoalkyl with 1 to 4 carbon atoms and 1 to 5 halogen atoms, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,

Z represents halogen, alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally substituted by halogen and/or alkyl with 1 to 4 carbon atoms and

m represents the number 0, 1, 2 or 3,

it being possible for the radicals Z to be identical or different, are used.

3. Process according to claim 1, characterised in that 2-tert.-butyl-2-(4-chloro-phenyl-ethyl)-oxirane is used as the oxirane of the formula (II).

4. Process according to claim 1, characterised in that compounds of the formula (IV)

in which

$R^1$ represents methyl or ethyl and

n represents the number 3, 4 or 5,

are used as the cyclic amids.

5. Process according to claim 1, characterised in that N-methyl-pyrrolidone, N-ethyl-pyrrolidone, N-methyl-hexahydropyridone or N-methyl-ε-caprolactam are used as the cyclic amides of the formula (IV).

6. Process according to claim 1, characterised in that alkali metal carbonates, alkali metal hydroxides, alkali metal alcoholates, alkali metal hydrides, lower tertiary alkylamines, cycloalkylamines or aralkylamines are used as the bases.

7. Process according to claim 1, characterised in that azo-bis-isobutyronitrile, benzoyl peroxide, air or oxygen are used as co-catalysts.

8. Process according to claim 1, characterised in that water is used as the auxiliary solvent.

9. Process according to claim 1, characterised in that the reaction is carried out at temperatures between 60 °C and 150 °C.